# EUROPEAN PATENT APPLICATION

(11) **EP 3 777 847 A1**
(43) Date of publication of application: **17.02.2021**
(21) Application number: 19774914.6
(22) Date of filing: 28.03.2019
(51) Int. Cl.: A61K 31/16, A61K 31/4164, A61P 13/12, A61P 11/00, A61P 1/16

(54) **PHARMACEUTICAL COMPOSITION FOR PREVENTION OR TREATMENT OF FIBROSIS**

(30) Priority: 28.03.2018 KR 20180035523; 27.03.2019 KR 20190034855
(71) Applicant: Crystalgenomics, Inc., Seongnam-si, Gyeonggi-do 13488 (KR)
(72) Inventor: CHO, Joong Myung, Seoul 05553 (KR); CHO, Jae Pyoung, Gunpo-si, Gyeonggi-do 15822 (KR); CHA, Hyunju, Seoul 06714 (KR); KIM, Young-Dae, Gwangju-si, Gyeonggi-do 12771 (KR)
(74) Representative: Schiweck Weinzierl Koch Patentanwälte Partnerschaft mbB
(86) International application number: PCT/KR2019/003619
(87) International publication number: WO 2019/190214

(57) **Abstract**

The present invention relates to a pharmaceutical composition comprising alkylcarbamoyl naphthalenyloxy octenoyl hydroxyamide, a derivative thereof, or a pharmaceutically acceptable salt thereof as an effective ingredient for prevention or treatment of fibrosis. Effectively suppressing the proliferation of fibrous tissues, the composition can be used for preventing and/or treating fibrosis.

## Description

### BACKGROUND OF THE INVENTION

### 1. Field of the Invention

The present invention relates to a pharmaceutical composition comprising alkylcarbamoyl naphthalenyloxy octenoyl hydroxyamide, a derivative thereof, or a pharmaceutically acceptable salt thereof as an effective ingredient for prevention or treatment of fibrosis.

### 2. Description of the Related Art

Fibrosis refers to excess of accumulation of fibrous connective tissue, which is due to the imbalance between proliferation and degradation of fibrous tissues. A common feature of these diseases is hyperproliferation of fibrotic cells, and the tissue or organ fibrosis often includes pulmonary fibrosis, hepatic fibrosis, chronic pancreatitis, scleroderma, glomerular fibrosis and multiple organ fibrosis caused by radiochemotherapy and tissue transplantation.

Renal fibrosis is a disease in which fibrosis occurs in scars accumulated due to inflammation in kidney tissue, in which a part of the kidney hardens and loses its function. This leads to chronic renal failure, which is accompanied by anemia, clotting disorders, hypertension, various complications and infections of cardiopulmonary organs and gastrointestinal tracts. If kidney function drops below 15 percent of normal, kidneys produce less erythropoietin, resulting in decreased production of red blood cells. In addition, uremia, which is caused by inactive urinary secretion, reduces red blood cell lifespan and causes strong anemia. In addition, the onset of uremia increases the likelihood of systemic infection, which is a major factor in the development of sepsis.

Idiopathic pulmonary fibrosis (IPF) is known as a representative disease that causes fibrosis within the lungs. It is known to be caused by an abnormality in the healing process of the damaged area caused by continuously stimulating epithelial cells or goblet cells, but its stimulating factor has not been known. It cannot be said that inflammation of the lung directly causes pulmonary fibrosis, but it is known that pulmonary fibrosis occurs due to a difference between a patient with idiopathic pulmonary fibrosis and a normal person in the process of healing to normal tissue following inflammation of the lungs. Another major mechanism of fibrosis is deposition and fibrosis of extracellular matrix through activation and proliferation of fibroblasts by T helper type 2 cytokines.

Hepatic fibrosis can be defined as excessive deposition of the extracellular matrix due to chronic inflammation in the liver. If chronic liver disease persists due to such excessive deposition of extracellular matrix, it eventually processes to cirrhosis due to distortion of the liver's internal structure a decrease in the number of liver cells. Representative cells involved in hepatic fibrosis include hepatic stellate cells, Kupffer cells, and endothelial cells. The activated hepatic stellate cells are the main source of extracellular matrix production and the hepatic stellate cells are involved in increasing the production of various extracellular matrix including collagen. Kupffer cells are located in the sinusoidal space in the liver, and substances produced by activated Kupffer cells affect surrounding hepatic cells, endothelial cells, and hepatic stellate cells, thereby promoting hepatic fibrosis. Endothelial cells not only play an essential role in the regulation of blood flow in the liver, but also are involved in the production of growth factors and extracellular matrix involved in the proliferation of hepatic stellate cells by inflammation or liver fibrosis. Cytokines affecting hepatic fibrosis include transforming growth factor-β (TGF-β) and platelet derived growth factor (PDGF). TGF-β is the most potent cytokine that promotes fibrosis of hepatic stellate cells, and hepatic stellate cells themselves are the main source of TGF-β. PDGF is the most potent cytokine that promotes division and proliferation of hepatic stellate cells. In the past, the hepatic fibrosis process has been recognized as an irreversible phenomenon, but recently, it has been reported to be reversibly changed. Thus, it can be a dynamic process. Therefore, it becomes clinically important to measure such change accurately.

The present inventors found that alkylcarbamoyl naphthalenyloxy octenoyl hydroxyamide having a specific formula, a derivative thereof, or a pharmaceutically acceptable salt thereof has an effect of inhibiting and improving fibrosis and completed the present invention.

### SUMMARY OF THE INVENTION

The present invention is aimed to provide a pharmaceutical composition comprising alkylcarbamoyl naphthalenyloxy octenoyl hydroxyamide, a derivative thereof, or a pharmaceutically acceptable salt thereof as an effective ingredient for prevention or treatment of fibrosis.

In order to solve the above problem, the present invention provides a pharmaceutical composition comprising a compound of the following formula 1, a derivative thereof, or a pharmaceutically acceptable salt thereof as an effective ingredient for prevention or treatment of fibrosis. 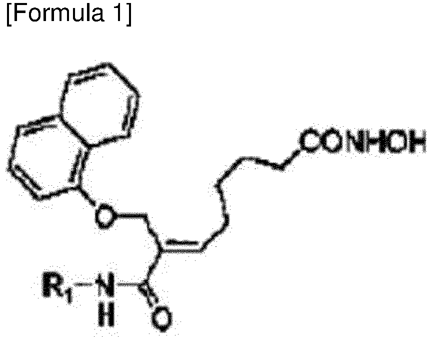 wherein, R₁ is C₁₋₃ alkyl which is unsubstituted or substituted with one or more substituents selected from the group consisting of halophenyl, C₁₋₃ alkoxy, C₁₋₃ alkoxy C₁₋₃ alkyl, cyclohexanyl, furanyl, thiophenyl, imidazole, imidazolidyl C₁₋₃ alkyl, C₁₋₃ alkylamino, di-C₁₋₃ alkylamino, hydroxyphenyl, tetrahydrofuranyl, cyclohexyl, cyclohexenyl, oxopyrrolidinyl, C₁₋₃ alkoxyphenyl, di-C₁₋₃ alkylaminophenyl, C₁₋₃ alkylpyrrolidinyl and trifluoromethoxyphenyl; pyrrolidine which is unsubstituted or substituted with C₃₋₈ cycloalkyl, C₃₋₈ cycloalkyl C₁₋₃ alkyl, benzyl, C₁₋₃ alkyl or C₃₋₈ cycloalkylcarbonyl; or piperidine substituted with C₁₋₃ alkyl or C₃₋₈ cycloalkyl; furan; or C₃₋₈ cycloalkyl, with the proviso that unsubstituted C₁₋₂ alkyl and C₁₋₂ alkyl substituted with C₁₋₂ alkylpyrrolidinyl are excluded.

In one embodiment of the present invention, the compound of the formula 1 is selected from the group consisting of the following compounds:
1) (E)-N1-(3-(1H-imidazol-1-yl)propyl)-N8-hydroxy-2-((naphthalen-1-yloxy)methyl)octendiam ide,
2) (E)-N8-hydroxy-N1-(4-hydroxyphenethyl)-2-((naphthalen-1-yl oxy) methyl)-2-octendiamide,
3) (E)-N1-(3-(dimethylamino)-2,2-dimethylpropyl)-N8-hydroxy-2-((naphthalen-1-yloxy)methyl)octendiamide,
4) (E)-N1-(2-(diisopropylamino)ethyl)-N8-hydroxy-2-((naphthalen-1-yloxy)methyl)octendiam ide,
5) (E)-N8-hydroxy-N1-(1-methoxypropan-2-yl)-2-((naphthalen-1-yloxy)methyl)-2-octendiamide,
6) (E)-N8-hydroxy-N1-(4-methoxybenzyl)-2-((naphthalen-1-yloxy)methyl)-2-octendiamide,
7) (E)-N1-(4-fluorophenethyl)-N8-hydroxy-2-((naphthalen-1-yloxy)methyl)-2-octendiamide,
8) (E)-N8-hydroxy-2-((naphthalen-1-yloxy)methyl)-N1-(tetrahydrofuran-2-yl)methyl)-2-octendiamide,
9) (E)-N1-(2-cyclohexenylethyl)-N8-hydroxy-2-((naphthalen-1-yloxy)methyl)-2-octendiamide,
10) (E)-N8-hydroxy-2-((naphthalen-1-yloxy)methyl)-N1-(3-(2-oxopyrrolidin-1-yl)propyl)-2-octendiamide,
11) (E)-N1-(furan-2-ylmethyl)-N8-hydroxy-2-((naphthalen-1-yloxy)methyl)-2-octendiamide,
12) (E)-N1-(4-(dimethylamino)benzyl)-N8-hydroxy-2-((naphthalen-1-yloxy)methyl)-2-octendiamide,
13) (E)-N8-hydroxy-N1-(2-methoxyethyl)-2-((naphthalen-1-yloxy)methyl)-2-octendiamide,
14) (E)-N1-cyclohexyl-N8-hydroxy-2-((naphthalen-1-yloxy)methyl)-2-octendiamide,
15) (E)-N8-hydroxy-2-((naphthalen-1-yloxy)methyl)-N1-(thiophen-2-ylmethyl)-2-octendiamide,
16) (E)-N8-hydroxy-N1-(4-methoxyphenethyl)-2-((naphthalen-1-yloxy)methyl)-2-octendiamide,
17) (E)-N8-hydroxy-2-((naphthalen-1-yloxy)methyl)-N1-(4-(trifluoromethoxy)benzyl)-2-octendiamide,
18) (E)-N1-(1-(cyclohexylmethyl)pyrrolidin-3-yl)-N8-hydroxy-2-((naphthalen-1-yloxy)methyl)-2-octendiamide,
19) (E)-N1-(1-cyclopentylpiperidin-4-yl)-N8-hydroxy-2-((naphthalen-1-yloxy)methyl)-2-octendiamide,
20) (E)-N1-(1-benzylpyrrolidin-3-yl)-N8-hydroxy-2-((naphthalen-1-yloxy)methyl)-2-octendiamide,
21) (E)-N8-hydroxy-N1-(1-isopropylpyrrolidin-3-yl)-2-((naphthalen-1-yloxy)methyl)-2-octendiamide,
22) (E)-N1-(1-(cyclohexanecarbonyl)pyrrolidin-3-yl)-N8-hydroxy-2-((naphthalen-1-yloxy)methyl)-2-octendiamide,
23) (E)-3-(8-(hydroxyamino)-2-((naphthalen-1-yloxy)methyl)-8-oxo-2-octenamido)pyrrolidine-1-carboxylic acid t-butyl ester,
24) (E)-N8-hydroxy-2-((naphthalen-1-yloxy)methyl)-N1-(pyrrolidin-3-yl)2-octendiamide,
25) (E)-N1-(1-cyclohexylpyrrolidin-3-yl)-N8-hydroxy-2-((naphthalen-2-yloxy)methyl)-2-octendiamide,
26) (E)-N1-(1-cyclopropylpyrrolidin-3-yl)-N8-hydroxy-2-((naphthalen-1-yloxy)methyl)-2-octendiamide,
27) (E)-N1-(1-cyclopropylpiperidin-4-yl)-N8-hydroxy-2-((naphthalen-1 - yloxy)methyl)-2-octendiamide;
28) (E)-N1-(1-ethylpiperidin-4-yl)-N8-hydroxy-2-((naphthalen-1-yloxy)methyl)-2-octendiamide,
29) (E)-N1-(1-ethylpyrrolidin-3-yl)-N8-hydroxy-2-((naphthalen-1-yloxy)methyl)-2-octendiamide,
30) (E)-N8-hydroxy-N1-(2-(1-methylpyrrolidin-2-yl)ethyl)-2-((naphthalen-1-yloxy)methyl)-2-octendiamide, and
31) (E)-N8-hydroxy-N1-(1-isopropylpiperidin-4-yl)-2-((naphthalen-1-yloxy)methyl)-2-octendiamide.

In one embodiment of the present invention, the compound of the formula 1 may be (E)-N1-(3-(dimethylamino)propyl)-N8-hydroxy-2-((naphthalen-1-yloxy)methyl)oct-2-enediamide represented by the following formula 2.

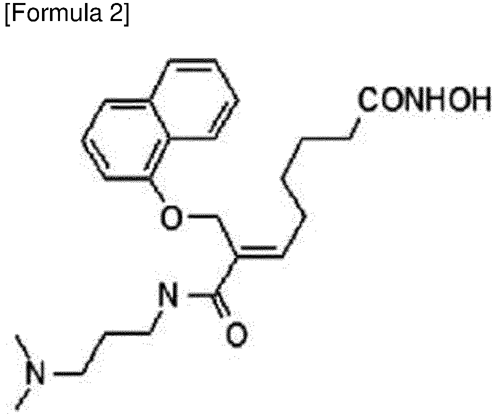

In one aspect of the present invention, the fibrosis may be renal fibrosis.

In another aspect of the present invention, the fibrosis may be pulmonary fibrosis, and the pulmonary fibrosis may be induced by humidifier disinfectants as well as a general bleomycin-induced model. The humidifier disinfectants include polyhexamethylene guanidine (PHMG), chloromethyl isothiazolinone (CMIT), methyl isothiazolinone (MIT), and oligo(2-(2-ethoxy)ethoxyethyl)guanidinium chloride (PGH).

In one aspect of the invention, the pulmonary fibrosis may be induced by one or more selected from the group consisting of bleomycin, polyhexamethylene guanidine (PHMG), chloromethyl isothiazolinone (CMIT), methyl isothiazolinone (MIT) and oligo(2-(2-ethoxy)ethoxyethyl)guanidinium chloride (PGH).

In another aspect of the invention, the fibrosis may be hepatic fibrosis.

In one aspect of the invention, the pharmaceutically acceptable salt may be one or more selected from the group consisting of phosphate, tartrate, stearate, gluconate, fumarate, naphthoate, and 1-hydroxy-2-naphthoic acid salt.

The pharmaceutical composition according to the present invention may be in the form of capsules, tablets, granules, injections, ointments, powders or beverages.

The pharmaceutical composition according to the present invention may be administered to human beings.

The pharmaceutical composition according to the present invention may be formulated in the form of oral dosage forms such as powders, granules, capsules, tablets and aqueous suspensions, external preparations, suppositories, and sterile injection solutions, respectively, according to a conventional method, but is not limited thereto. The pharmaceutical composition of the present invention may comprise a pharmaceutical acceptable carrier. As the pharmaceutically acceptable carrier, binders, lubricants, disintegrants, excipients, solubilizers, dispersants, stabilizers, suspending agents, pigments, fragrances, etc. may be used for oral administration, buffers, preservatives, soothing agents, solubilizers, isotonic agents, stabilizers, etc. may be used for injections, and bases, excipients, lubricants, preservatives, etc. may be used for topical administration. The formulation of the pharmaceutical composition of the present invention can be prepared in various ways in combination with the pharmaceutical acceptable carrier as described above. For example, for oral administration, tablets, troches, capsules, elixirs, suspensions, syrups, wafers, etc. may be prepared, and in the case of injections, unit dosage ampoules or multiple dosage forms may be prepared. In addition, it can be formulated into solutions, suspensions, tablets, capsules, sustained-release preparations, etc.

The routes of administration of the pharmaceutical composition according to the present invention is oral, intravenous, intramuscular, intraarterial, intramedullary, intrathecal, intracardiac, transdermal, subcutaneous, intraperitoneal, intranasal, intestinal, topical, sublingual or rectal. Oral or parenteral administration is preferred. The term "parenteral" as used herein refers to subcutaneous, intradermal, intravenous, intramuscular, intraarticular, intrasynovial, intrasternal, intradural, intralesional and intracranial injection or infusion techniques. The pharmaceutical composition of the present invention may also be administered in the form of suppositories for rectal administration.

The pharmaceutical composition according to the present invention may be administered to mammals, such as rats, mice, livestock, and humans by various routes. Any modes of administration can be expected, for example, oral, rectal or intravenous, intramuscular, subcutaneous, intrabronchial inhalation, intrauterine or intracerebroventricular injections.

The pharmaceutical composition of the present invention may vary depending on a variety of factors, including the activity of the specific compound used, age, weight, general health, sex, diet, administration time, administration route, discharge rate, drug combination and severity of the specific disease to be prevented or treated. The dosage of the pharmaceutical composition may vary depending on the health condition, weight, disease severity of the subject, and the route and duration of administration, but may be appropriately selected by a person skilled in the art, and may be administered at 0.0001 to 50 mg/kg or 0.001 to 50 mg/kg per day. Administration may be administered once a day, or may be divided into several times. The above dosage does not limit the scope of the invention in any way. The pharmaceutical composition according to the present invention may be formulated as pills, dragees, capsules, liquids, gels, syrups, slurries, suspensions.

The dosage of the compound of formula 1, which is an effective ingredient of the pharmaceutical composition according to the present invention, may vary depending on the age, sex, weight, and degree of disease of the subject, but may be 0.01 to 100 mg/kg, and preferably 0.1 to 50 mg/kg once to several times daily.

In addition, the dosage of the compound of formula 1 according to the present invention may be increased or decreased depending on the route of administration, the degree of disease, sex, weight, and age of the subject. Therefore, the above dosage does not limit the scope of the present invention in any way.

### EFFECT OF THE INVENTION

The pharmaceutical composition according to the present invention inhibits expression of α-SMA (alpha-smooth muscle actin), a fibrotic marker of cells and shows anti-fibrotic effects of tissues in an animal model of renal fibrosis induced by acute renal failure and an animal model of pulmonary fibrosis induced by bleomycin, polyhexamethylene guanidine (PHMG), etc, thereby enabling to effectively treat or prevent long-term fibrosis.

### BRIEF DESCRIPTION OF THE DRAWINGS

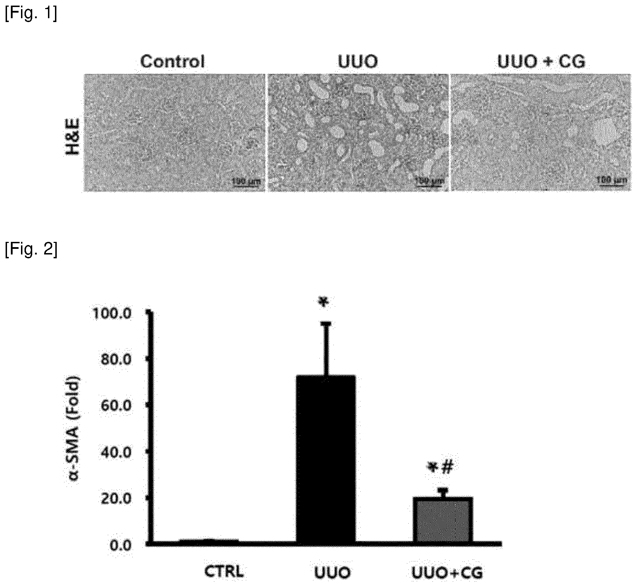

Fig. 1 is a histopathological analysis result from hematoxylin and eosin staining on kidney tissue section of the CG200745 intake group in which 30 mg/kg/day of (E)-N1-(3-(dimethylamino)propyl)-N8-hydroxy-2-((naphthalene-1-yloxy)methyl)oct-2-enediamide (compound of formula 2, hereinafter referred to as "CG200745") is consumed and the solvent control group in an animal model of renal fibrosis induced by acute renal failure and on the left kidney tissue section of the animal model of normal kidney as a negative control.
Fig. 2 is a graph comparing expression levels of α-SMA (alpha-smooth muscle actin) by performing real-time polymerase chain reaction of RNA isolated from the kidney tissue cells of the CG200745 intake group in which 30 mg/kg/day of CG200745 is consumed and the solvent control group in an animal model of renal fibrosis induced by acute renal failure and from the left kidney tissue cells of the animal model of normal kidney as a negative control.

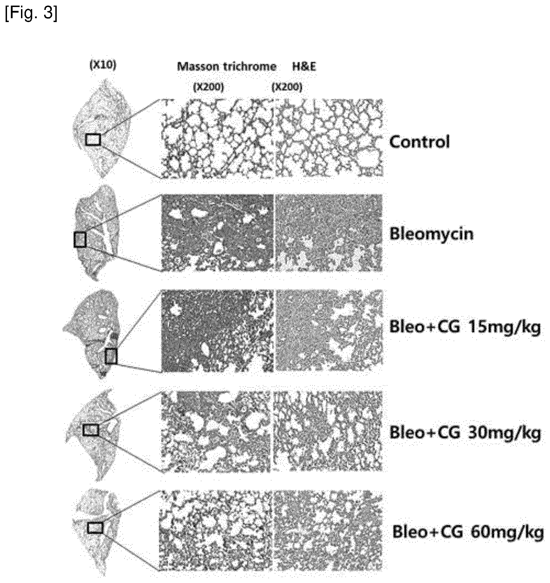

Fig. 3 is a lung tissue section and a histopathological analysis result from hematoxylin and eosin staining of the CG200745-administered group in which CG200745 is administered at 15, 30, 60 mg/kg/day or the solvent control group in an animal model of pulmonary fibrosis induced by bleomycin.

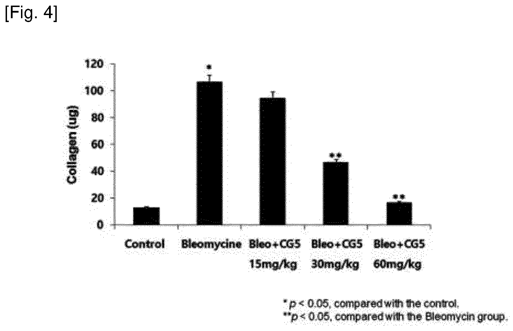

Fig. 4 is a graph comparing the concentration of collagen by performing sircol assay with bronchoalveolar lavage fluid of the CG200745-administered group in which CG200745 is administered at 15, 30, 60 mg/kg/day or the solvent control group in an animal model of pulmonary fibrosis induced by bleomycin.

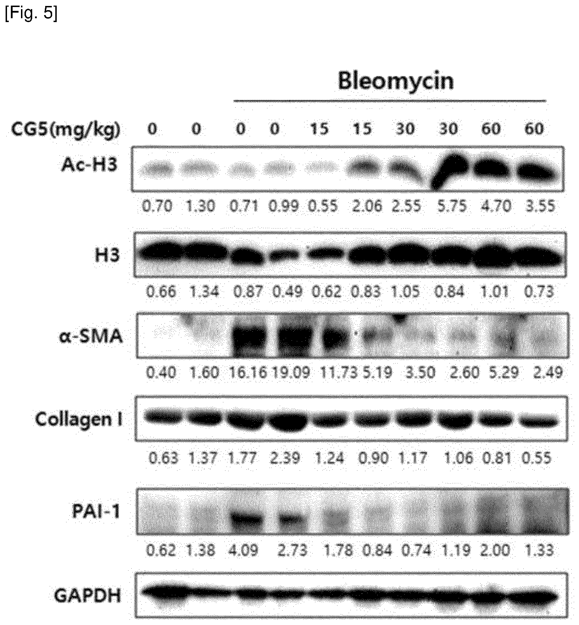

Fig. 5 is a result of verification of expression levels of target proteins for fibrosis of lung tissue cells of the CG200745-administered group in which CG200745 is administered at 15, 30, 60 mg/kg/day or the solvent control group in an animal model of pulmonary fibrosis induced by bleomycin.

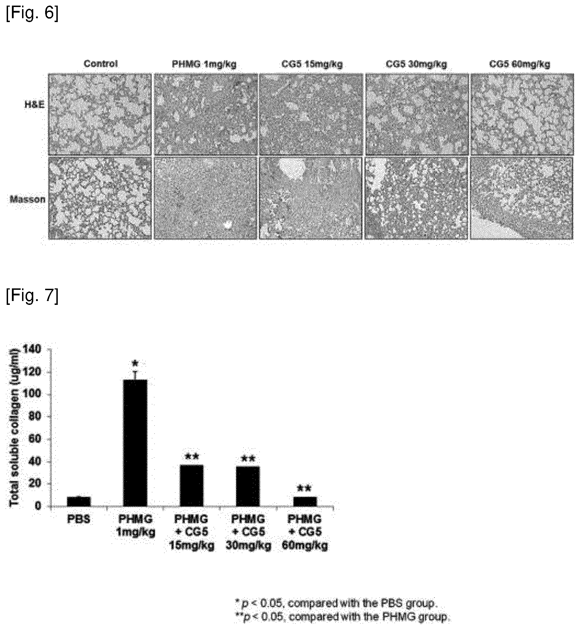

Fig. 6 is a lung tissue section and a histopathological analysis result from hematoxylin and eosin staining and Masson's trichrome staining of the CG200745-administered group in which CG200745 is administered at 15, 30, 60 mg/kg/day or the solvent control group in an animal model of pulmonary fibrosis induced by PHMG.
Fig. 7 is a graph comparing the concentration of collagen by performing sircol assay with bronchoalveolar lavage fluid (BALF) of the CG200745-administered group in which CG200745 is administered at 15, 30, 60 mg/kg/day or the solvent control group in an animal model of pulmonary fibrosis induced by PHMG.

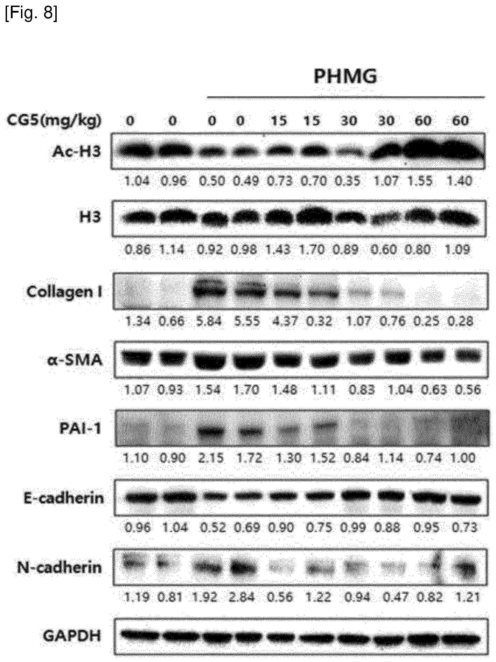

Fig. 8 is a result of verification of expression levels of target proteins for fibrosis of lung tissue cells of the CG200745 administration group in which CG200745 is administered at 15, 30, 60 mg/kg/day or the solvent control group in an animal model of pulmonary fibrosis induced by PHMG.

### DETAILED DESCRIPTION OF THE INVENTION

Since various modifications and variations can be made in the present invention, particular embodiments are illustrated in the drawings and will be described in detail in the detailed description. It should be understood, however, that the invention is not intended to be limited to the particular embodiments, but includes all modifications, equivalents, and alternatives falling within the spirit and scope of the invention. In the following description of the present invention, detailed description of known functions will be omitted if it is determined that it may obscure the gist of the present invention.

### <Example 1> Evaluation of unilateral ureteral obstruction (renal failure) mouse model

### 1-1. Production of unilateral ureteral obstruction mouse model

8-week old female C57BL/6 mice were used. After opening the abdomen of C57BL/6 mice, one of two ureters was closed with 6-0 cotton yarn. After closing the ureter, the abdomen was sutured and disinfected. Immediately after surgery, the host mice were classified into the solvent control group which is the non-intake group, and the CG200745 intake group in which CG200745 dissolved in distilled water was consumed (30 mg/kg/day). On the 7th day after surgery, the solvent control group and the CG200745 intake group mice were euthanized, and then normal kidney and the kidney with acute renal failure induced after ureteral obstruction were extracted.

### 1-2. Histopathology analysis

The extracted normal kidney and kidney with acute renal failure induced were fixed with 4% paraformaldehyde, and then covered with paraffin to prepare a tissue section with a 3 µm thickness. Hematoxylin and eosin staining was conducted on the completed tissue sections to perform a histopathological analysis of renal tissues. For hematoxylin staining, kidney tissue sections were stained with Gill's hematoxylin for 5 minutes, washed with tap water, and then treated with 95% ethanol. Eosin staining was performed by staining with eosin and phloxine for 1 minute. The tissue sections were then dehydrated with ethanol and xylene and fixed with Canada balsam. The prepared kidney tissue sections were observed with an optical microscope. As a result, as shown in Fig 1, the fibrosis progressed in the solvent control group which is the kidney with acute renal failure induced, compared to the negative control which is normal kidney, whereas the fibrosis progression was inhibited in the kidney of the CG200745 intake group compared to the solvent control group.

### 1-3. Verification of expression level of fibrosis marker α-SMA (alpha-smooth muscle actin)

RNA was isolated from the extracted normal kidney and kidneys of the solvent control group and CG200745 intake group which are the kidney with acute renal failure induced, to synthesize cDNA by reverse transcription, and real-time polymerase chain reaction (real-time PCR) was performed to compare expression levels of α-SMA (alpha-smooth muscle actin).

The kidney cortex was homogenized with Trizol solution (Invitrogen, Carlsbad, CA). RNA was extracted from the homogenized kidney cortex using chloroform, precipitated in isopropanol, and washed with 75% ethanol. The washed RNA was dissolved in distilled water and then the concentration was determined by measuring absorbance at 260 nm (Ultraspec 2000; Pharmacia Biotech, Cambridge, UK). cDNA was produced by reverse transcription of 5 mg RNA using oligo(dT) primer and superscript reverse transcriptase II (Invitrogen, Carlsbad, CA). The produced cDNA was quantified by measuring with SYBR Green using Smart Cycler II System (Cepheid, Sunnyvale, CA).

For real-time polymerase chain reaction, 10 mM forward primer, 10 mM reverse primer, 2X SYBR Green Premix Ex Taq (TAKARA BIO INC, Seta 3-4-1, Japan) and 0.5 mL cDNA were mixed and then distilled water was added so that the final volume was 20 mL. Real-time polymerase chain reaction was performed using Rotor-GeneTM 3000 Detector System (Corbette research, Mortlake, New South Wales, Australia) to compare the relative quantification of α-SMA and mRNA expression.

The real-time polymerase chain reaction was performed in the following steps.
1) 95°C, 5 min
2) 95 °C, 20 sec
3) 58 ∼ 62 °C, 20 sec
4) 72 °C, 30 sec
5) 85 °C, 6 sec

Of these, steps 2) to 5) were repeatedly performed 64 times, and the final temperature of the last cycle was increased from 60 °C to 95 °C to generate a melting curve. PCR expression level comparison was confirmed by SYBR Green measurement. The following primer sequences were used:
Human α-SMA forward, 5'-ACTGGGACGACATGGAAAAG-3' and reverse, 5'-CATCTCCAGAGTCCAGCACA-3', Human GAPDH forward, 5'-TGTGTCCGTCGTGGATCTGA-3' and reverse, 5-GATGCCTGCTTCACCACCTT-3'.

As a result, as shown in Fig 2, it is found that α-SMA expression was reduced by 50% or more in the CG200745 intake group in which 30 mg/kg of CG200745 is consumed in the kidney with renal failure induced through ureteral obstruction, compared to the solvent control group which is the non-intake group.

### <Example 2> Evaluation of bleomycin-induced mouse model

### 2-1. Production of bleomycin-induced mouse model

As a bleomycin model, 7-week old female C57BL/6 mice were used, and the mice were divided into a total of 5 groups, a bleomycin-administered group, CG200745-administered groups in which CG200745 is administered at 15, 30, 60 mg/kg after administration of bleomycin, and a control group to perform the experiment. Airway incision was performed on the mice and 2 mg/kg bleomycin was injected into the airway in a volume of 50 µl. After injection, the incision site was closed with 5-0 nylon. After the injection of bleomycin, the drug was administered intraperitoneally for a total of 2 weeks.

### 2-2. Histopathological analysis in a bleomycin-induced mouse model

The mice were sacrificed and subjected to chest opening and then the lungs were removed and fixed with 4% paraformaldehyde. They were fixed by immersing in 4% paraformaldehyde for 48 hours at 4°C, and then a paraffin block was prepared. 4 µm of microsection was made from the paraffin-embedded tissue and attached to a slide. Hemotoxin and eosin staining and Masson's trichrome staining were performed.

For hemotoxin and eosin staining, the slide was immersed in xylene twice for 5 minutes each and in 100%, 95% and 70% ethanol for 2 minutes each, and washed with running distilled water. After washing, the slide was immersed in hematoxylin for 1 minute, washed several times with running distilled water, then immersed in eosin for 30 seconds and washed several times with running distilled water. Then, the slide was immersed in 70%, 95%, and 100% ethanol for 1 minute each and in xylene twice for 2 minutes each. Finally, a cover-slide was permanently attached using mounting medium xylene and the sample was observed through an optical microscope.

For Masson's trichrome staining to dye collagen and muscle fibers, the slide was immersed in xylene twice for 5 minutes each and in 100%, 95% and 70% ethanol for 2 minutes each, and washed with running distilled water. After washing, the slide was reacted in Bouin's fluid for 1 hour in a constant temperature water bath set at 56 to 64°C, and then cooled for 10 minutes. After washing the slide several times with distilled water, it was stained in iron hematoxylin working solution for 5 minutes, and rinsed for 2 minutes in warm water, and washed once again with distilled water. It was stained in Biebrich scarlet/Acid fuchsin solution for 5 minutes and then washed with distilled water. It was stained in Phosphomolybdic phosphotungstic acid solution for 15 minutes and then washed with distilled water. It was stained in Anilin Blue solution for 10 minutes, washed with distilled water and immersed in 1% acetic acid solution for 5 minutes. Then, it was immersed in 70%, 95% and 100% ethanol for 1 minute each and in xylene twice for 2 minutes each. Dyed collagen and muscle fibers were observed through an optical microscope.

As a result, as shown in Fig. 3, it was found that the degree of fibrosis and collagen accumulation which had been increased by bleomycin treatment was significantly decreased in CG200745 concentration-dependent manner and normalized.

### 2-3. Sircol assay in bleomycin-induced mouse model

After two weeks of drug administration, the skin of the airway of the mouse was incised to expose the airway and washed with 800 µl of PBS into the trachea and then 500 µl of the solution was recovered therefrom to obtain bronchoalveolar lavage fluid. The bronchoalveolar lavage fluid was centrifuged at 4°C and 1,200 rpm for 5 minutes to obtain supernatant. 50 µl of the bronchoalveolar lavage fluid and 1 ml of sircol dye reagent were mixed for 30 minutes. After centrifugation at 4°C and 12,000 rpm for 10 minutes, the supernatant was discarded, 750 µl of Acid-salt wash reagent was added for washing, and then centrifugation was performed at 12,000 rpm for 10 minutes. After discarding the supernatant, 1 ml of Alkali reagent was added and absorbance was measured at 555 nm.

As a result, as shown in Fig. 4, it was found that collagen which had been increased by bleomycin treatment was significantly decreased in CG200745 concentration-dependent manner.

### 2-4. Verification of expression of fibrosis marker in bleomycin-induced mouse models

Western blot was performed to identify makers of pulmonary fibrosis (α-SMA, collagen I, PAI-1, E-cadherin, N-cadherin) and Acetylated H3 in tissues. The mice were sacrificed, and then subjected to chest opening and the lungs were removed and stored frozen for a day, and the mouse tissues were ground to prepare a protein sample. It was subjected to SDS-PAGE and the fractionated protein sample was transferred to PVDF (polyvinylidene difluoride) membrane. The PVDF membrane transferred with the proteins was blocked by shaking for 1 hour in blocking buffer diluted with 5% skim milk in 1X TBST, and then polyclonal primary antibodies derived from rabbit were diluted 1000 times in blocking buffer and reacted overnight. After the reaction was completed, the membrane was washed with buffer and anti-rabbit secondary antibodies conjugated to HRP were diluted 2000 times in blocking buffer and reacted for 1 hour. After washing the membrane with buffer, reaction with ECL solution was conducted on the washed membrane.

With the average for two controls being 1, the relative value was recorded to conduct densitometry analysis. As a result, as shown in Fig. 5, it was found that the level of histone acetylation was increased and the expression levels of fibrosis markers (a-SMA, Collagen I, PAI-1) which had been increased by bleomycin treatment were reduced in CG200745 concentration-dependent manner.

### <Example 3> Evaluation of polyhexamethylene guanidine-induced mouse model

### 3-1. Production of polyhexamethylene guanidine-induced pulmonary fibrosis mouse model

As a polyhexamethylene guanidine (PHMG) model, 7-week old female C57BL/6 mice were used, and the mice were divided into a total of 5 groups, a polyhexamethylene guanidine-administered group, CG200745-administered groups in which CG200745 is administered at 15, 30, 60 mg/kg after administration of polyhexamethylene guanidine, and a control group to perform the experiment. Airway incision was performed on the mice and 1 mg/kg polyhexamethylene guanidine was injected into the airway in a volume of 50 µl. After injection, the incision site was closed with 5-0 nylon. After the injection of polyhexamethylene guanidine, the drug was administered intraperitoneally for a total of 2 weeks.

### 3-2. Histopathological analysis in a polyhexamethylene guanidine-induced pulmonary fibrosis mouse model

The mice were sacrificed and subjected to chest opening and then the lungs were removed and fixed with 4% paraformaldehyde. They were fixed by immersing in 4% paraformaldehyde for 48 hours at 4°C, and then a paraffin block was prepared. 4 µm of microsection was made from the paraffin-embedded tissue and attached to a slide. Hemotoxin and eosin staining and Masson's trichrome staining were performed.

For hemotoxin and eosin staining, the slide was immersed in xylene twice for 5 minutes each and in 100%, 95% and 70% ethanol for 2 minutes each, and washed with running distilled water. After washing, the slide was immersed in hematoxylin for 1 minute, washed several times with running distilled water, then immersed in eosin for 30 seconds and washed several times with running distilled water. Then, the slide was immersed in 70%, 95%, and 100% ethanol for 1 minute each and in xylene twice for 2 minutes each. Finally, a cover-slide was permanently attached using mounting medium xylene and the sample was observed through an optical microscope.

For Masson's trichrome staining to dye collagen and muscle fibers, the slide was immersed in xylene twice for 5 minutes each and in 100%, 95% and 70% ethanol for 2 minutes each, and washed with running distilled water. After washing, the slide was reacted in Bouin's fluid for 1 hour in a constant temperature water bath set at 56 to 64°C, and then cooled for 10 minutes. After washing the slide several times with distilled water, it was stained in iron hematoxylin working solution for 5 minutes, and rinsed for 2 minutes in warm water, and washed once again with distilled water. It was stained in Biebrich scarlet/Acid fuchsin solution for 5 minutes and then washed with distilled water. It was stained in Phosphomolybdic phosphotungstic acid solution for 15 minutes and then washed with distilled water. It was stained in Anilin Blue solution for 10 minutes, washed with distilled water and immersed in 1% acetic acid solution for 5 minutes. Then, it was immersed in 70%, 95% and 100% ethanol for 1 minute each and in xylene twice for 2 minutes each. Dyed collagen and muscle fibers were observed through an optical microscope.

As a result, as shown in Fig. 6, it was found that polyhexamethylene guanidine-induced pulmonary fibrosis was suppressed in CG200745 concentration-dependent manner and normalized.

### 3-3. Sircol assay in a polyhexamethylene guanidine-induced pulmonary fibrosis mouse model

After two weeks of drug administration, the skin of the airway of the mouse was incised to expose the airway and washed with 800 µl of PBS into the trachea and then 500 µl of the solution was recovered therefrom to obtain bronchoalveolar lavage fluid. The bronchoalveolar lavage fluid was centrifuged at 4°C and 1,200 rpm for 5 minutes to obtain supernatant. 50 µl of the bronchoalveolar lavage fluid and 1 ml of sircol dye reagent were mixed for 30 minutes. After centrifugation at 4°C and 12,000 rpm for 10 minutes, the supernatant was discarded, 750 µl of Acid-salt wash reagent was added for washing, and then centrifugation was performed at 12,000 rpm for 10 minutes. After discarding the supernatant, 1 ml of Alkali reagent was added and absorbance was measured at 555 nm.

As a result, as shown in Fig. 7, it was found that collagen which had been increased by polyhexamethylene guanidine was significantly decreased in CG200745 concentration-dependent manner.

### 3-4. Verification of expression of fibrosis marker in a polyhexamethylene guanidine-induced pulmonary fibrosis mouse model

Western blot was performed to identify makers of pulmonary fibrosis (α-SMA, collagen I, PAI-1, E-cadherin, N-cadherin) and Acetylated H3 in tissues. The mice were sacrificed, and then subjected to chest opening and the lungs were removed and stored frozen for a day, and the mouse tissues were ground to prepare a protein sample. It was subjected to SDS-PAGE and the fractionated protein sample was transferred to PVDF (polyvinylidene difluoride) membrane. The PVDF membrane transferred with the proteins was blocked by shaking for 1 hour in blocking buffer diluted with 5% skim milk in 1X TBST, and then polyclonal primary antibodies derived from rabbit were diluted 1000 times in blocking buffer and reacted overnight. After the reaction was completed, the membrane was washed with buffer and anti-rabbit secondary antibodies conjugated to HRP were diluted 2000 times in blocking buffer and reacted for 1 hour. After washing the membrane with buffer, reaction with ECL solution was conducted on the washed membrane.

With the average for two controls being 1, the relative value was recorded to conduct densitometry analysis. As a result, as shown in Fig. 8, it was found that the level of histone acetylation was increased and the expression levels of fibrosis markers (a-SMA, Collagen I, PAI-1) which had been increased by polyhexamethylene guanidine treatment were reduced in CG200745 concentration-dependent manner.

The pulmonary fibrosis model can be induced by one or more selected from the group consisting of bleomycin, polyhexamethylene guanidine, chloromethyl isothiazolinone, methyl isothiazolinone, and oligo(2-(2-ethoxy)ethoxyethyl)guanidinium chloride in addition to the components described above.

The specific parts of the present invention have been described in detail above. For those skilled in the art, this specific technique is only a preferred embodiment, and it is obvious that the scope of the present invention is not limited thereto. Therefore, the substantial scope of the present invention will be defined by the appended claims and their equivalents.

## Claims

1. A pharmaceutical composition comprising a compound of the following formula 1, a derivative thereof, or a pharmaceutically acceptable salt thereof as an effective ingredient for prevention or treatment of fibrosis. 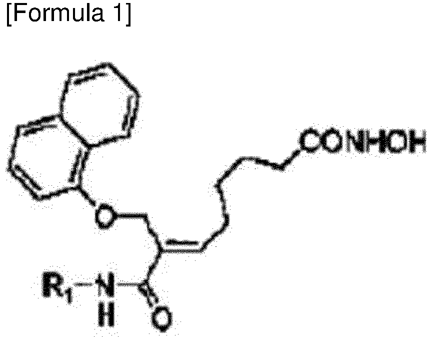 wherein, R₁ is C₁₋₃ alkyl which is unsubstituted or substituted with one or more substituents selected from the group consisting of halophenyl, C₁₋₃ alkoxy, C₁₋₃ alkoxy C₁₋₃ alkyl, cyclohexanyl, furanyl, thiophenyl, imidazole, imidazolidyl C₁₋₃ alkyl, C₁₋₃ alkylamino, di-C₁₋₃ alkylamino, hydroxyphenyl, tetrahydrofuranyl, cyclohexyl, cyclohexenyl, oxopyrrolidinyl, C₁₋₃ alkoxyphenyl, di-C₁₋₃ alkylaminophenyl, C₁₋₃ alkylpyrrolidinyl and trifluoromethoxyphenyl; pyrrolidine which is unsubstituted or substituted with C₃₋₈ cycloalkyl, C₃₋₈ cycloalkyl C₁₋₃ alkyl, benzyl, C₁₋₃ alkyl or C₃₋₈ cycloalkylcarbonyl; or piperidine substituted with C₁₋₃ alkyl or C₃₋₈ cycloalkyl; furan; or C₃₋₈ cycloalkyl, with the proviso that unsubstituted C₁₋₂ alkyl and C₁₋₂ alkyl substituted with C₁₋₂ alkylpyrrolidinyl are excluded.

2. The pharmaceutical composition according to claim 1, wherein the compound of the formula 1 is selected from the group consisting of the following compounds:
1) (E)-N1-(3-(1H-imidazol-1-yl)propyl)-N8-hydroxy-2-((naphthalen-1-yloxy)methyl)octendiam ide,
2) (E)-N8-hydroxy-N1-(4-hydroxyphenethyl)-2-((naphthalen-1-yl oxy)methyl)-2-octendiamide,
3) (E)-N1-(3-(dimethylamino)-2,2-dimethylpropyl)-N8-hydroxy-2-((naphthalen-1-yloxy)methyl)octendiamide,
4) (E)-N1-(2-(diisopropylamino)ethyl)-N8-hydroxy-2-((naphthalen-1-yloxy)methyl)octendiam ide,
5) (E)-N8-hydroxy-N1-(1-methoxypropan-2-yl)-2-((naphthalen-1-yloxy)methyl)-2-octendiamide,
6) (E)-N8-hydroxy-N1-(4-methoxybenzyl)-2-((naphthalen-1-yloxy)methyl)-2-octendiamide,
7) (E)-N1-(4-fluorophenethyl)-N8-hydroxy-2-((naphthalen-1-yloxy)methyl)-2-octendiamide,
8) (E)-N8-hydroxy-2-((naphthalen-1-yloxy)methyl)-N1-(tetrahydrofuran-2-yl)methyl)-2-octendiamide,
9) (E)-N1-(2-cyclohexenylethyl)-N8-hydroxy-2-((naphthalen-1-yloxy)methyl)-2-octendiamide,
10) (E)-N8-hydroxy-2-((naphthalen-1-yloxy)methyl)-N1-(3-(2-oxopyrrolidin-1-yl)propyl)-2-octendiamide,
11) (E)-N1-(furan-2-ylmethyl)-N8-hydroxy-2-((naphthalen-1-yloxy)methyl)-2-octendiamide,
12) (E)-N1-(4-(dimethylamino)benzyl)-N8-hydroxy-2-((naphthalen-1-yloxy)methyl)-2-octendiamide,
13) (E)-N8-hydroxy-N1-(2-methoxyethyl)-2-((naphthalen-1-yloxy)methyl)-2-octendiamide,
14) (E)-N1-cyclohexyl-N8-hydroxy-2-((naphthalen-1-yloxy)methyl)-2-octendiamide,
15) (E)-N8-hydroxy-2-((naphthalen-1-yloxy)methyl)-N1-(thiophen-2-ylmethyl)-2-octendiamide,
16) (E)-N8-hydroxy-N1-(4-methoxyphenethyl)-2-((naphthalen-1-yloxy)methyl)-2-octendiamide,
17) (E)-N8-hydroxy-2-((naphthalen-1-yloxy)methyl)-N1-(4-(trifluoromethoxy)benzyl)-2-octendiamide,
18) (E)-N1-(1-(cyclohexylmethyl)pyrrolidin-3-yl)-N8-hydroxy-2-((naphthalen-1-yloxy)methyl)-2-octendiamide,
19) (E)-N1-(1-cyclopentylpiperidin-4-yl)-N8-hydroxy-2-((naphthalen-1-yloxy)methyl)-2-octendiamide,
20) (E)-N1-(1-benzylpyrrolidin-3-yl)-N8-hydroxy-2-((naphthalen-1 - yloxy)methyl)-2-octendiamide,
21) (E)-N8-hydroxy-N1-(1-isopropylpyrrolidin-3-yl)-2-((naphthalen-1-yloxy)methyl)-2-octendiamide,
22) (E)-N1-(1-(cyclohexanecarbonyl)pyrrolidin-3-yl)-N8-hydroxy-2-((naphthalen-1-yloxy)methyl)-2-octendiamide,
23) (E)-3-(8-(hydroxyamino)-2-((naphthalen-1-yloxy)methyl)-8-oxo-2-octenamido)pyrrolidine-1-carboxylic acid t-butyl ester,
24) (E)-N8-hydroxy-2-((naphthalen-1-yloxy)methyl)-N1-(pyrrolidin-3-yl)2-octendiamide,
25) (E)-N1-(1-cyclohexylpyrrolidin-3-yl)-N8-hydroxy-2-((naphthalen-2-yloxy)methyl)-2-octendiamide,
26) (E)-N1-(1-cyclopropylpyrrolidin-3-yl)-N8-hydroxy-2-((naphthalen-1-yloxy)methyl)-2-octendiamide,
27) (E)-N1-(1-cyclopropylpiperidin-4-yl)-N8-hydroxy-2-((naphthalen-1 - yloxy)methyl)-2-octendiamide;
28) (E)-N1-(1-ethylpiperidin-4-yl)-N8-hydroxy-2-((naphthalen-1-yloxy)methyl)-2-octendiamide,
29) (E)-N1-(1-ethylpyrrolidin-3-yl)-N8-hydroxy-2-((naphthalen-1-yloxy)methyl)-2-octendiamide,
30) (E)-N8-hydroxy-N1-(2-(1-methylpyrrolidin-2-yl)ethyl)-2-((naphthalen-1-yloxy)methyl)-2-octendiamide, and
31) (E)-N8-hydroxy-N1-(1-isopropylpiperidin-4-yl)-2-((naphthalen-1-yloxy)methyl)-2-octendiamide.

3. The pharmaceutical composition according to claim 1, wherein the compound of the formula 1 is (E)-N1-(3-(dimethylamino)propyl)-N8-hydroxy-2-((naphthalen-1-yloxy)methyl)oct -2-enediamide.

4. The pharmaceutical composition according to claim 1, wherein the fibrosis is renal fibrosis.

5. The pharmaceutical composition according to claim 1, wherein the fibrosis is pulmonary fibrosis.

6. The pharmaceutical composition according to claim 5, wherein the pulmonary fibrosis is induced by one or more selected from the group consisting of bleomycin, polyhexamethylene guanidine, chloromethyl isothiazolinone, methyl isothiazolinone and oligo(2-(2-ethoxy)ethoxyethyl)guanidinium chloride.

7. The pharmaceutical composition according to claim 1, wherein the fibrosis is hepatic fibrosis.

8. The pharmaceutical composition according to claim 1, wherein the pharmaceutically acceptable salt is one or more selected from the group consisting of phosphate, tartrate, stearate, gluconate, fumarate, naphthoate, and 1-hydroxy-2-naphthoic acid salt.

9. The pharmaceutical composition according to claim 1, wherein the pharmaceutical composition is in the form of capsules, tablets, granules, injections, ointments, powders or beverages.
